# EUROPÄISCHE PATENTSCHRIFT

(11) **EP 2 091 429 B1**
(45) Veröffentlichungstag und Bekanntmachung des Hinweises auf die Patenterteilung: **10.11.2010**
(21) Anmeldenummer: 07816254.2
(22) Anmeldetag: 14.11.2007
(51) Int. Cl.: A61B 5/085

(54) **VERFAHREN UND VORRICHTUNG ZUR BESTIMMUNG DES PEEP BEI DER BEATMUNG EINES PATIENTEN**
METHOD AND DEVICE FOR DETERMINING THE PEEP DURING THE RESPIRATION OF A PATIENT
PROCÉDÉ ET DISPOSITIF POUR DÉTERMINER LA PEEP AU COURS DE LA RESPIRATION D'UN PATIENT

(30) Priorität: 16.11.2006 CH 18432006
(43) Veröffentlichungstag der Anmeldung: 26.08.2009
(73) Patentinhaber: Hamilton Medical AG, 7402 Bonaduz (CH)
(72) Erfinder: WYSOCKI, Marc, 78460 Chevreves (FR); LAUBSCHER, Thomas, 7403 Rhäzüns (CH); DURISCH, Gion, 7013 Domat/Ems (CH); BRUNNER, Josef, 7000 Chur (CH)
(74) Vertreter: Hasler, Erich
(86) Internationale Anmeldenummer: PCT/CH2007/000571
(87) Internationale Veröffentlichungsnummer: WO 2008/058417

(56) Entgegenhaltungen:
- EP-A1- 0 671 180
- WO-A-03/037413
- US-A1- 2003 111 078
- US-A1- 2004 073 130

## Beschreibung

Ein PEEP (Positive EndExpiratory Pressure) bezeichnet einen bei der Beatmung künstlich in der Lunge erzeugten positiven Druck, der nach Abschluss der Ausatmung (Exspiration) anliegt. Dieser Druck erhöht den Atemwegsmitteldruck und die funktionelle Residualkapazität. Ein PEEP kann dazu beitragen, einen Kollaps der Lungenbläschen zu verhindern. Mittels eines PEEP kann in vielen Fällen die Sauerstoffsättigung des Blutes verbessert werden. Aus diesen Gründen wird bei der maschinellen Beatmung oft zumindest ein mäßiger PEEP verwendet.

Das Beatmen mit einem PEEP kann jedoch auch Nachteile haben. Durch die Erhöhung des Drucks im Brustraum verringert PEEP den Rückfluss des venösen Blutes zum Herzen, wodurch das Herzzeitvolumen sinken kann. Umgekehrt entsteht ein Rückstau in die obere und die untere Hohlvene mit entsprechenden Druckerhöhungen in vorgeschalteten Organen. Abhängig von der Höhe des PEEP kann es dadurch zu Schädigungen und Funktionseinschränkungen von Gehirn, Leber, Nieren u.a. Organe kommen.

Moderne Beatmungsgeräte bieten detaillierte Einstell- und Messmöglichkeiten zur PEEP-Beatmung. Bei angepasster Einstellung des Druckes werden die Nachteile des positiven Druckes in der Ausatemphase (Gefahr des Barotraumas bei zu hohem PEEP-Niveau, Gefahr der Schädigung gesunder Lungenareale, Erhöhte rechtsventrikuläre Nachlast, Zunahme des intrakraniellen Drucks) durch die Vorteile (Vergrößerung der funktionellen Residualkapazität, dadurch Erhöhung der Gasaustauschfläche, Ventilations- Perfusion-Verhältnis, verminderte intrapulmonale Shunts, erhöhte Oxygenierung, Vermeidung von Atelektase, Verbesserung der Compliance, Reduktion eines Lungenödems) aufgewogen.

Es ist Aufgabe der Erfindung, eine Vorrichtung zu schaffen, mit der ein angemessener PEEP automatisch eruiert werden kann.

Eine diese Aufgabe erfindungsgemäss lösende Vorrichtung ist durch die Merkmale des Anspruchs 1 gekennzeichnet.

Die erfindungsgemässe Vorrichtung zum automatisierten Ermitteln eines auf einen Patienten abgestimmten PEEP umfasst Sensoren und eine geeignete Elektronik. Mit diesen Sensoren und der die Sensormesswerte verarbeitenden Elektronik kann bei einem P/V-Manöver, also während dem ein Beatmungsdruck erhöht und wieder reduziert wird - oder von einem hohen Druckniveau reduziert und danach wieder erhöht wird -, eine Druck-Volumen-Kurve ermittelt werden. Eine solche Druck-Volumenkurve kann mit jedem der dafür geeigneten, bekannten Verfahren und mit an sich bekannten Vorrichtungen ermittelt werden. Es wird hier diesbezüglich auf den Stand der Technik verwiesen, der in der WO 03/037413 und auch in der DE 10 2005 000 964 B3 beschrieben ist. Weitere Verfahren sind in den Dokumente US 2004/073130 A1, US 2003/111078 A1 und EP 0 671180 A1 zu finden.

US 2004/073130 offenbart eine Vorrichtung zur Bestimmung der alveolären Öffnung und Schliessung der Lunge mittels elektrischer Impedanztomographiemessungen, wobei die Vorrichtung Mittel zur Messung von Impedanzsignalen in Abhängigkeit des Beatmungsdrucks beinhaltet.

Zweckmässigerweise wird das dabei in die Lunge des Patienten einströmende Volumen zu einem jeweiligen Beatmungsdruck zugeordnet aufaddiert und das ausströmende Volumen wieder subtrahiert. Die Differenz gibt etwa das Volumen an, das durch den Beatmungsdruck rekrutiert wurde. Die Elektronik ist ausgelegt, um beatmungsdruckspezifisch die Differenz aus den Lungenvolumina zu bilden. Es wird Beatmungsdruckspezifisch jeweils vom Lungenvolumen beim Ausatmen ("deflation": Vdef) das Lungenvolumen beim Einatmen ("insufflation": Vinf) abgezogen. Die Elektronik ist ferner ausgelegt, um ein Differenzmaximum zu bestimmen, und einen Wert des Beatmungsdrucks zu ermitteln, der an einer in Relation zum Maximum definierten Stelle der Differenzkurve herrscht. Es wird in der Folge aufgrund des ermittelten Werts des Beatmungsdrucks ein Wert für PEEP errechnet. Eine zweckmässige Ermittlung dieser relevanten Stelle der Differenzkurve geschieht, indem ein Betrag vom Maximum der Differenz abgezogen wird, und die Stellen gesucht werden, die diese reduzierte Differenz aufweisen. Von den in der Regel zwei solchen Stellen, wird diejenige genommen, an der ein tieferer Beatmungsdruck herrscht als beim Maximum.

Eine erfindungsgemässe Ermittlung des für den Patienten angemessenen PEEP aufgrund des Beatmungsdruckes, bei welchem die Volumendifferenz Vdef-Vinf nahe am Maximum ist, hat den Vorteil, dass dieser Wert des Beatmungsdruckes sehr einfach und automatisiert errechnet werden kann.

Die Vorrichtung ist zweckmässigerweise mit einem Display ausgerüstet, auf dem der ermittelte Wert für den PEEP angezeigt werden kann. Eine von einem Beatmungsgerät unabhängige Vorrichtung ist mit eigenen Sensoren ausgestattet. Diese können beispielsweise in die Beatmungsschläuche eines beatmeten Patienten eingefügt werden. Mit dem Beatmungsgerät kann dann z.B. eine Druckrampe von 0 bis 40 mbar und zurück gefahren werden. Dabei wird der PEEP automatisch bestimmt und angezeigt. Ein Arzt braucht diesen lediglich noch zu übernehmen und dem Beatmungsgerät einzugeben. Anstelle einer Druckrampe kann auch ein Fluss gesteuert werden. Es kann beispielsweise ein konstanter Fluss generiert werden. Dabei ist jedoch ebenfalls das Volumen dem Druck zugeordnet zu erfassen.

Die Vorrichtung kann jedoch auch die Sensoren eines Beatmungsgeräts nutzen, um die für die Berechnung des PEEP notwendigen Messungen durchzuführen.

Eine an ein Beatmungsgerät gebundene, oder in dieses integrierte Vorrichtung nutzt selbstverständlich die Sensoren des Beatmungsgeräts und auch den Display des Beatmungsgeräts. Die Sensoren können in diesem Fall im Beatmungsgerät oder im Beatmungsschlauch integriert sein. Die Vorrichtung kann auch derart in das Beatmungsgerät integriert sein, dass der ermittelte Wert automatisch vom Beatmungsgerät übernommen wird.

Eine vorteilhafte Vorrichtung weist daher eine elektronische Verbindung zu einem Beatmungsgerät oder dessen Steuerung auf. Diese Verbindung dient der Übertragung des ermittelten Werts für PEEP an den Display und/ oder bevorzugst an die Steuerung des Beatmungsgeräts.

Die Erfindung geht davon aus, dass der ermittelte Wert für den Beatmungsdruck, bei dem die Volumendifferenz am grössten oder nahezu am grössten ist, bereits sehr gut einem dem Patienten angemessenen PEEP entspricht. Er kann daher direkt als einzustellender PEEP übernommen werden. Es ist jedoch nicht auszuschliessen, dass zumindest in gewissen Fällen eine bessere Einstellung erreicht wird, wenn der gemessene Druck noch rechnerisch angepasst wird. Dafür kann vorgesehen sein, dass bei der Vorrichtung Mittel vorhanden sind, um einem Krankheitsbild und/ oder einem Körperindex des Patienten entsprechend unterschiedliche Zahlenwerte aus einer Tabelle zu wählen oder zu berechnen. Die Elektronik ist dann derart ausgelegt, dass sie zur Bestimmung des PEEP diesen Wert des Beatmungsdrucks mit dem durch erwähnte Mittel gewählten oder berechneten Zahlenwert verrechnet, z.B. potenziert, multipliziert oder um den gewählten oder berechneten Zahlenwert korrigiert.

Vorteilhaft wird nicht in jedem Fall ein PEEP oder erhöhter PEEP errechnet. Es ist deshalb ein Grenzwert für die Volumendifferenz festgelegt, und die Elektronik ermittelt nur dann einen PEEP oder einen erhöhten PEEP, wenn die ermittelte Volumendifferenz oberhalb dieses Grenzwerts liegt. Unterhalb dieser Grenze liegende Volumendifferenzen zeigen sodann an, dass der PEEP auf dem bisherigen Wert belassen oder eventuell sogar gesenkt werden kann.

Dieser Grenzwert ist zweckmässigerweise in Abhängigkeit eines Körperindexes des Patienten bemessen. Als Körperindex kann beispielsweise die Körperlänge oder ein Idealgewicht verwendet werden. Weitere Faktoren, die bei der Festlegung des Grenzwerts berücksichtigt werden können, oder vorteilhaft sollen, sind das Krankheitsbild des Patienten und das Alter des Patienten, bzw. ob der Patient Kind oder erwachsen ist.

Die Vorrichtung ist zweckmässigerweise mit einer Zeitmesseinrichtung versehen. Diese erlaubt die Steuerung von zeitlichen Abläufen. Die Vorrichtung ist vorteilhaft so ausgerüstet, dass sie den Anstieg und den Abfall des Beatmungsdruckes steuern und in zeitlichen Abständen wiederholt eine Bestimmung eines neuen PEEP auslösen kann. Sie kann dazu im Beatmungsgerät integriert sein oder eigene Mittel zur Generierung eines Druckanstiegs und - abfalles besitzen.

Die Vorrichtung kann mit einem Beatmungsgerät so kombiniert sein, dass das Beatmungsgerät den ermittelten Wert für PEEP automatisch übernimmt. Dies ist auch dann möglich, wenn die Vorrichtung nicht Teil des Beatmungsgeräts ist. Zweckmässig ist jedoch in Beatmungsgeräten mit automatisierter Anpassung der Beatmung an die Bedürfnisse des Patienten eine solche Vorrichtung zu integrieren.

Eine solche Vorrichtung arbeitet nach folgendem Erfahren. :
Zum automatischen Ermitteln eines auf einen Patienten abgestimmten PEEP wird
1. während der Durchführung eine P/V-Manövers eine Druck-Volumen-Kurve ermittelt. Diese wird ermittelt indem z.B. mit dem Beatmungsdruck eine Rampe hochgefahren und wieder hinunter gefahren wird, oder indem ein konstanter Fluss generiert wird, und das dabei eingeatmete Volumen dem jeweiligen Druck zugeordnet aufaddiert und das ausgeatmete Volumen dem jeweiligen Druck zugeordnet vom eingeatmeten Volumen wieder subtrahiert wird.
2. Dann wird bei der so ermittelten Druck-Volumen-Kurve das Maximum der Volumendifferenz (Vdef -Vinf)max ermittelt.
3. Sodann wird ein Wert für den Beatmungsdruck ermittelt, der an einer in Relation zum Maximum definierten Stelle der Differenzkurve gilt,
4. und schliesslich dieser ermittelte Wert des Beatmungsdrucks als Ausgangswert für eine Festlegung des PEEP genommen.

In der Praxis kann sich ergeben, das die Kurve, die die Differenz darstellt, keinen eindeutigen Scheitelpunkt hat. Es wird deshalb vorgeschlagen, dass der angemessene PEEP ermittelt wird, indem ein Maximum gesucht wird, von diesem ausgehend ein unterer Schwellwert festgelegt wird (gewählt oder errechnet in Abhängigkeit von Alter, Grösse und/ oder Krankheitsbild der beatmeten Person, z.B. 5, 10, 20 oder 50 ml unter dem Maximum), und schliesslich der tiefere der beiden Drücke dem PEEP zugrunde gelegt wird, welche an den Stellen herrschen, an denen die Differenz der Volumina diesen unteren Schwellwert aufweist. In der Regel werden sich zwei solche Stellen ergeben, nämlich eine mit einem höheren Druck als beim Maximum und die gesuchte, bei der ein niedrigerer Druck als beim Maximum herrscht. Der niedrigere Wert des Beatmungsdruckes wird zum Ausgangspunkt für die PEEP-Berechnung, oder wird als PEEP genommen.

Es ist auch möglich, dass sich mehrere solche Stellen ergeben. Es kann dann der niedrigere der beiden an das Maximum angrenzenden Werte genommen werden, oder es kann der niedrigste Wert genommen werden.

Wird das Maximum der Differenz erst nach Durchlaufen des gesamten P/V-Manövers bestimmt, so kann sowohl das Maximum der Volumendifferenz als auch der niedrigste Wert des dem PEEP zugrunde zu legenden Beatmungsdrucks eindeutig bestimmt werden.

Diese Art der Festlegung des relevanten Beatmungsdruckes erlaubt aber auch ein Abbrechen des P/V-Manövers bei Erreichen des gesuchten Beatmungsdrucks. Zweckmässigerweise wird dazu zuerst der Druck in der Lunge aufgebaut und danach wieder reduziert. Während dem Reduzieren kann laufend die Differenz druckspezifisch errechnet werden. Solange diese Differenz ansteigt, gilt jeweils der neue Differenzwert als Maximum. Beim Abfallen der Werte gilt jeweils der höchste Wert als Maximum, und zwar solange, bis ein höherer Wert ermittelt wird. Fällt dann das Volumen ab bis auf einen definierten Schwellwert unterhalb des Maximums, so ist der dann herrschende Druck Ausgangswert zur PEEP-Bestimmung oder direkt der Druck für die zukünftige PEEP-Einstellung.

Es ist theoretisch möglich, dass diese Wert zu hoch liegt, weil später, bei geringerem Beatmungsdruck, noch ein höheres Maximum gemessen worden wäre. Wird jedoch der Betrag zwischen dem Maximum und dem unteren Schwellwert genügend gross genommen, so kann ein solcher erneuter Anstieg der Volumendifferenz praktisch ausgeschlossen werden.

Dieser ermittelte Wert kann direkt als PEEP genommen werden oder auch, wenn erforderlich, rechnerisch korrigiert werden. Dieser rechnerische Korrekturwert wird z.B. einem Krankheitsbild und/ oder einem Körperindex entsprechend gewählt. Dadurch kann individuell auf den Patienten und seine Bedürfnisse Rücksicht genommen werden. Falls ein PEEP berechnet wird, kann die Druckdifferenz, innerhalb welcher die Volumendifferenzwerte innerhalb der erwähnten Bandbreite liegen, in die Berechnung mit einfliessen.

Vorteilhaft wird ein Grenzwert für die Volumendifferenz festgelegt. Falls die ermittelte Volumendifferenz oberhalb dieses Grenzwerts liegt wird ein neuer PEEP ermittelt, bzw. der aktuelle PEEP dem Resultat entsprechend erhöht. Falls die ermittelte Volumendifferenz unterhalb dieses Grenzwerts liegt wird der alte PEEP beibehalten oder eine PEEP-Reduktion vorgenommen. Eine PEEP-Reduktion aufgrund des Nicht-Erreichens dieses Grenzwerts erübrigt sich, wenn bei der verwendeten Druck-Volumenkurve der Druck zwischen Null und beispielweise 40 mbar variiert wurde. In diesem Fall kann bei einer maximalen Volumendifferenz, die unterhalb des Grenzwerts liegt, der PEEP auf einen vorgegebenen Minimalwert gesetzt werden. Wird jedoch der Grenzwert überschritten, gilt der neu gefundene Wert des Beatmungsdruckes bei der maximalen Volumendifferenz als PEEP oder als Grundlage für die PEEP-Bestimmung.

Es kann vorgesehen sein, dass das Ausmass einer Erhöhung des PEEP bzw. einer Reduktion des PEEP durch Maximalwerte begrenzt ist. Es werden dann grössere PEEP-Veränderungen in zwei oder mehr zeitlich von einander beabstandeten Schritten vorgenommen. Solche Maximalwerte der PEEP-Veränderung bleiben beispielsweise bei +5 mbar, (möglicherweise auch +8 oder +10) mbar und bei -2 oder -3 mbar, (möglicherweise auch -5 oder -8 mbar).

Eine PEEP-Bestimmung kann jeweils vom medizinischen Personal ausgelöst werden. Das Verfahren kann auch automatisiert in zeitlichen Abständen wiederholt werden, um den jeweils aktuellen PEEP festzustellen.

Bei einer automatisierten Wiederholung des Verfahrens gibt es zwei grundsätzlich unterschiedliche Vorgehensweisen. Es wird bei einer ersten Variante die Druckrampe unterhalb des eingestellten PEEP, insbesondere bei Null begonnen. Dies hat den Vorteil, dass auch ein tieferer als der gegenwärtige PEEP eruiert werden kann. Der Ausgangspunkt kann bei Null oder bei einem Zwischenwert zwischen Null und dem eingestellten PEEP gewählt sein.

Bei einer zweiten Variante wird die Druckrampe bei dem davor eingestellten PEEP begonnen. Diese Variante hat den Vorteil, dass keine Kollabierung von Lungenbläschen in Kauf genommen werden muss. Sie hat den Nachteil, dass kein tieferer PEEP ermittelt werden kann. Es kann indes vorgesehen sein, dass bei nicht Erreichen eines tieferen, zweiten Grenzwerts der Volumendifferenz eine Reduktion des PEEP vorgenommen wird.

Wird das Verfahren in Kombination mit einer mechanischen Beatmung durchgeführt, so kann der ermittelte Wert für PEEP vorteilhaft automatisch am Beatmungsgerät eingestellt werden. Dadurch ist eine automatische Regelung des PEEP erreicht.

Bei einer Erhöhung des PEEP wird vorteilhaft ein Recruitment-Manöver ausgeführt und danach mit dem ermittelten höheren PEEP beatmet. Dies ist insbesondere dann zweckmässig, wenn bei der Ermittlung des neuen PEEP-Werts der Beatmungsdruck bis unter den bisherigen PEEP abgesenkt wird.

Es kann jedoch auch vorgesehen werden, beim Ausatmen eine Druckrampe zu fahren und die Reduktion des Beatmungsdruckes sofort zu stoppen, sobald das Volumendifferenzmaximum ermittelt wurde. Anschliessend wird mit dem neu ermittelten PEEP beatmet.

Kurzbeschreibung der Figuren:
- Fig. 1: zeigt schematisch eine erste P/V-Schlaufe mit grosser Differenz zwischen Vdef und Vinf.
- Fig. 2: zeigt schematisch die Differenzbildung zwischen Vdef und Vinf für die erste P/V-Schlaufe.
- Fig. 3: zeigt schematisch eine zweite P/V-Schlaufe mit kleiner Differenz zwischen Vdef und Vinf.
- Fig. 4: zeigt schematisch die Differenzbildung zwischen Vdef und Vinf für die zweite P/V-Schlaufe.
- Fig. 5: zeigt eine erste Differenzbildung von an einem Menschen gemessenen Werten.
- Fig. 6: zeigt eine zweite Differenzbildung von an einem anderen Menschen gemessenen Werten.
- Fig. 7: zeigt ein Flussdiagramm für die Bestimmung des PEEP anhand des P/V-Loops.
- Fig. 8: zeigt eine von einem Beatmungsgerät unabhängige Vorrichtung zur Bestimmung des PEEP in der erfindungsgemässen Weise.
- Fig. 9: zeigt ein Beatmungsgerät mit einer darin integrierten, erfindungsgemässen Vorrichtung.

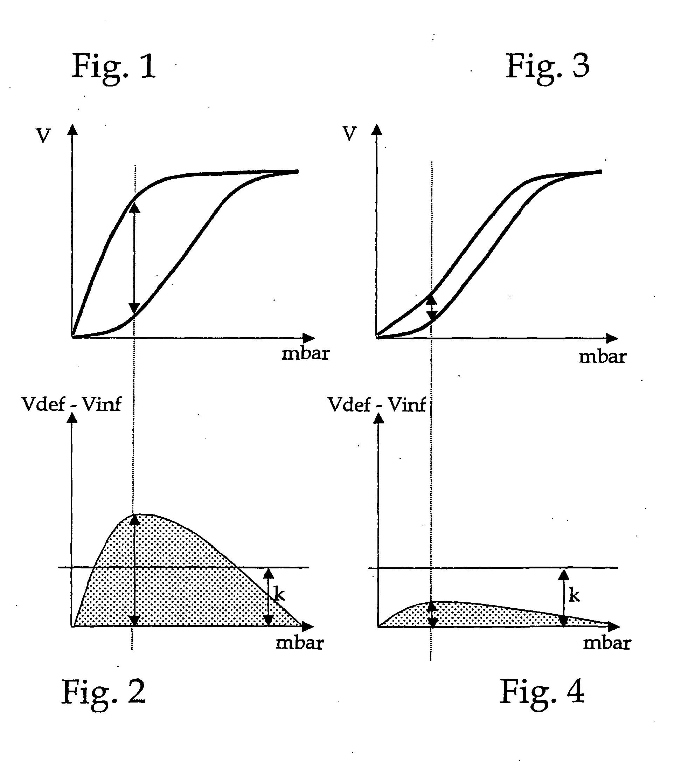

Anhand der in den Figuren 1 bis 4 dargestellten Diagramme wird im Folgenden die Erfindung im Detail beschrieben. Figur 1 zeigt eine P/V-Schlaufe eines Patienten, bei dem die Lunge besser mit einem PEEP beatmet wird. Der untere aufsteigende Ast wird gebildet, in dem die Lunge aufgebläht wird und jeweils das Volumen der eingeblasenen Luft gegenüber dem herrschenden Druck aufgezeichnet wird. Der obere absteigende Ast wird erfasst währenddem Luft aus der Lunge entweicht und so der Druck abgebaut wird. Dabei wird das in der Lunge verbleibende Luftvolumen in Relation zum herrschenden Druck aufgezeichnet, Die grosse Differenz zwischen dem aufsteigenden unteren Ast und dem absteigenden oberen Ast lässt darauf schliessen, dass Lungenbläschen bei geringem Beatmungsdruck kollabiert sind, aber bei höherem Beatmungsdruck geöffnet sind.

Für den Arzt stellt sich die Frage, bei welchem - möglichst tiefen - PEEP die einmal rekrutierten Lungenbläschen geöffnet bleiben. Es wurde gefunden, dass der angemessene PEEP sich in einem Diagramm gemäss Figur 1 an der Schlaufe ablesen lässt. Der angemessen PEEP ist der Druck, bei dem die grösste Volumendifferenz zwischen Vdef und Vinf bemessen wird.

Diese Stelle kann durch eine Differenzbildung zwischen Vdef und Vinf ermittelt werden. Die gleiche Stelle ist bei einem rekrutierten Volumen (Volumendifferenz) mit lediglich einem Peak definiert durch den Druck, bei welchem die Steigung der Vdef- und der Vinf-Kurve identisch ist. Es können daher auch die Tangenten an die Kurven gesucht und der Druck ermittelt werden, bei welchem die Tangenten an den aufsteigenden und den absteigenden Ast parallel sind. Einfacher und sicherer ist jedoch die Differenzbildung zwischen dem absteigenden und dem aufsteigenden Ast. Auch bei Kurven mit zwei Scheitelpunkten erhält man mit dieser Differenzbildung in aller Regel einen einzigen Wert für den Beatmungsdruck, bei dem die Differenz Vdef-Vinf ein Maximum erreicht.

Diese Differenzbildung ist zudem sehr einfach zu rechnen und daher ist der Scheitelpunkt der Kurve gemäss Figur 2 - und mit diesem der gewünschte Wert für den PEEP - sehr rasch ermittelt. Der Druck, bei dem der Scheitelpunkt in Figur 2 liegt, definiert den Druck, der - als PEEP verwendet - geeignet ist, die Lungenbläschen geöffnet zu halten.

Es kann sich bei weiteren Versuchen zeigen, dass es notwendig ist, die gemessene Kurve zu glätten, um einen eindeutigen Scheitelpunkt feststellen zu können. Es kann sich weiter zeigen, dass es sich positiv auswirkt, zu diesem, beim Scheitelpunkt wirkenden Druck eine Druckreserve von beispielsweise 2mbar hinzu zu fügen, um einen geeigneten PEEP zu erhalten, oder dass ein optimaler PEEP sogar etwas gegenüber dem beim Scheitelpunkt herrschenden Druck reduziert werden kann.

In Figuren 3 und 4 sind die selben Parameter dargestellt wie in Figuren 1 und 2. Die Messungen ergaben aber eine relativ geringe Differenz zwischen Vdef und Vinf. Eine Rekrutierung von zusätzlichen Alveolen ist hier nicht möglich. Bei einem solchen Patienten, ist es deshalb nicht notwendig und nicht angezeigt, einen erhöhten PEEP zu verwenden.

Es gibt daher, und dies ist aus den Figuren 2 und 4 ersichtlich, einen Grenzwert für die Differenz Vdef-Vinf. Dieser Grenzwert hat den Betrag k. Der jeweilige Wert von k kann mit Vorteil abhängig vom Patienten (Körperindex) und seinem Krankheitsbild festgelegt sein.

Hat die ermittelte Volumendifferenz einen maximalen Wert, der kleiner als k ist, so wird keine Korrektur des PEEP vorgenommen.

Lediglich bei einer Differenz, deren maximaler Wert grösser als k ist, wird ein PEEP festgesetzt.

Diese Regel kann auch auf eine PEEP-Korrektur angewendet werden, bei der ein P/V-Loop lediglich für die Drücke über dem eingestellten PEEP ermittelt wird. Hier kann ebenfalls der Grenzwert k angesetzt werden, um abzuklären, ob eine PEEP-Erhöhung zweckmässig ist. Dieser Grenzwert k liegt erfahrungsgemäss bei einem Erwachsenen durchschnittlich um 500 ml.

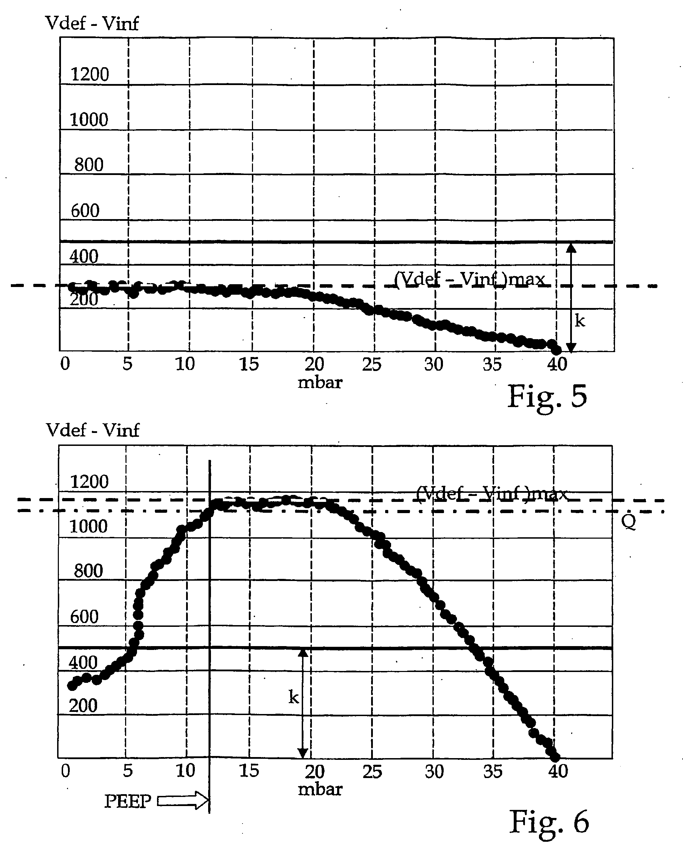

In Figuren 5 und 6 sind die Volumendifferenz- Diagramme von echten Messungen dargestellt. Die Figur 5 zeigt das Ergebnis einer Lunge, bei der eine Erhöhung des PEEP nicht zu einer Rekrutierung von Alveolen führt. Die Figur 6 zeigt ein Ergebnis einer Lunge, bei der ein PEEP um 12 mbar notwendig ist, um die meisten Lungenbläschen geöffnet zu halten. Aus dieser Figur 6 ist erkennbar, dass eine Glättung der Kurve hilfreich sein kann, um einen eindeutigen Scheitelpunkt feststellen zu können.

In Figur 6 ist ferner ein unterer Schwellwert (Q) in einem definierten Abstand zum Scheitelpunkt von (Vdef-Vinf) vorhanden. Der Beatmungsdruck, welcher der Ermittlung des PEEP zugrundegelegt wird, wird erst dann ermittelt, wenn die Differenz der Volumen diesen Schwellwert erreicht. Es kann auch der PEEP aufgrund dieses Beatmungsdruckes und des Beatmungsdruckes beim Scheitelpunkt errechnet werden (z.B. unterer Druck plus 1/3 von (oberer Druck minus unterer Druck).

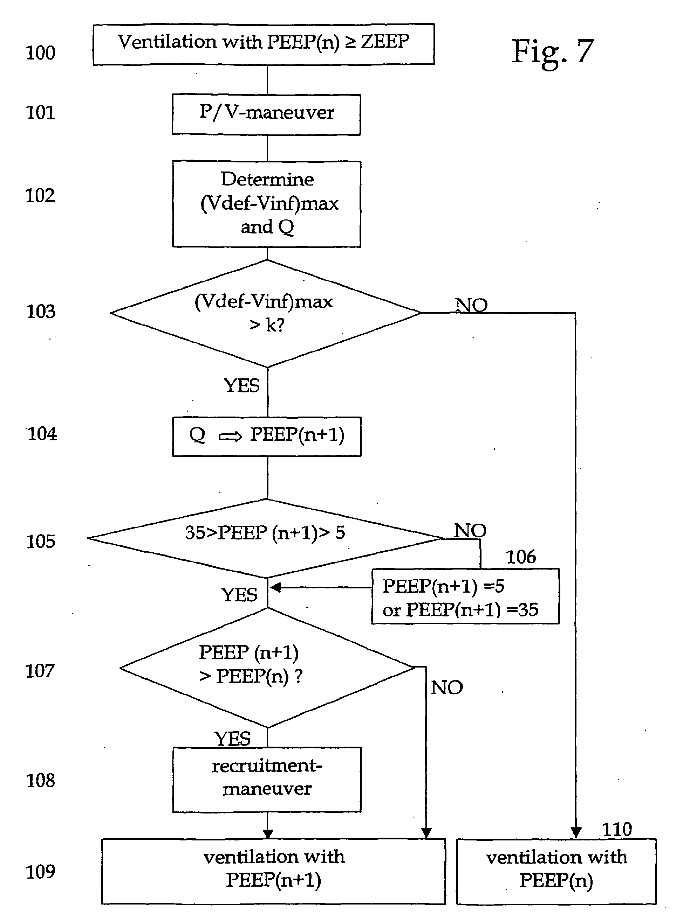

Das in Figur 7 dargestellte Flussdiagramm illustriert den Ablauf beim Bestimmen des geeigneten PEEP. Es wird von einer Beatmung mit einem beliebigen PEEP ausgegangen (Block 100). Der endexspiratorische Druck kann vor einer solchen PEEP-Bestimmung auch Null sein. Nun wird ein P/V-Manöver durchgeführt (Block 101). Dazu wird nach einer aus mehreren wählbaren Arten der Druck und das Luftvolumen in der Lunge erhöht und anschliessend wieder reduziert. Während diesem Vorgang wird das Lungenvolumen in Relation zum Druck aufgezeichnet. Der dabei verwendete obere Wert des verwendeten Druckes liegt beispielsweise bei 40 mbar. Der unterste Wert kann bei 0, zwischen Null und PEEP(n) oder beim bisherigen PEEP(n) liegen.

Anschliessend oder zeitgleich wird die Differenz des Lungenvolumens errechnet und die maximale Differenz bestimmt (Block 102). Mit dem Differenzmaximum ist auch der Schwellwert Q bestimmt. Ist die maximale Volumendifferenz (oder der Schwellwert Q) bestimmt, wird entschieden (Rhombus 103), ob diese maximale Volumendifferenz (oder der Schwellwert Q) den Grenzwert k übersteigt oder nicht. Ist die ermittelte Volumendifferenz kleiner als k, so wird mit dem bisherigen endexspiratorischen Druck weiter beatmet (Block 110) oder der PEEP reduziert. Die Ablaufvarianten für ein Reduzieren des PEEP sind in der Figur 7 nicht dargestellt.

Eine Reduktion des PEEP ist insbesondere dann angezeigt, wenn die P/V-Schlaufe lediglich mit Drücken ab dem bisher verwendeten PEEP durchgeführt wurde. Kriterien zur PEEP-Reduktion können sein: Wert des eingestellten PEEP, Zeit seit letzter PEEP-Erhöhung, Ausmass der letzten PEEP-Erhöhung, Krankheitsbild, Alter und Körperindex des Patienten, Anzahl der PEEP-Bestimmungen ohne Erhöhung des PEEP.

Eine automatisierte PEEP-Reduktion kann vermieden werden, wenn das P/V-Manöver mit Beatmungsdrücken unter dem gegenwärtig verwendeten PEEP beginnend (oder gar von Null weg) ausgeführt wird. Das so erhaltene Resultat ist unabhängig vom früher verwendeten PEEP.

Es ist deshalb sinnvoll das P/V-Manöver in zeitlichen Intervallen (z.B. stündlich, 3-stündlich, 6-stündlich, täglich 1 bis 2 mal) durchzuführen. Dabei kann z.B. so lange ein P/V-Manöver vom bisherigen PEEP ausgehend ausgeführt werden, als PEEP dabei jeweils erhöht zu werden braucht. Sollte dabei der auf erfindungsgemässe Art ermittelte angemessene PEEP nach mehreren (z.B. 3, 5 oder 8) Bestimmungen immer gleich bleiben, so kann danach bei der Bestimmung des PEEP ein P/V-Manöver ausgehend von einem tieferen Druck als dem gegenwärtigen PEEP(n) ausgeführt werden (z.B. zwei Drittel oder die Hälfte von PEEP(n)). Falls dabei der neue PEEP(n+1) tiefer liegt als der bisherige PEEP(n), so wird selbstverständlich dieser neue PEEP(n+1) bei der weiteren Beatmung verwendet. Auf diese Art kann die Häufigkeit des Kollabierens und erneuten Rekrutierens der Lungenbläschen reduziert und der Wert des PEEP bei Bedarf dennoch nach unten korrigiert werden.

Zurück zu Figur 7. In Block 104 ist dargestellt, dass aufgrund des Ortes des Schwellwerts Q auf der Differenzkurve der neue PEEP(n+1) ermittelt wird. Dieser neue PEEP kann in der einfachsten Ausführung der Druck sein, bei dem das Differenzmaximum oder der Druck Q festgestellt wurde. Er kann auch davon abweichen, ist aber sicher von einem dieser Drücke ausgehend berechnet.

Eine nächste Entscheidung ist die, festzustellen, ob der ermittelte Druck über einem ersten oder unter einem zweiten Grenzwert liegt und entsprechend auf den jeweiligen Grenzwert genommen werden muss (Rhombus 105). Ist der ermittelte Druck für PEEP z.B. 3 mbar, so wird PEEP(n+1) auf den unteren Grenzwert von beispielsweise 5mbar gesetzt (Block 106). Liegt er über beispielsweise 35 mbar, so wird er auf diesen Wert gesetzt.

In der Praxis wird sich weisen, ob eine solcher Grenzwert überhaupt notwendig ist. Es kann beispielsweise lediglich einseitig ein unterer Grenzwert notwendig sein, weil eine obere Begrenzung schon durch die obere Begrenzung des Druckes beim P/V-Loop erreicht ist.

Wird jedoch festgestellt (Rhombus 107), dass der neue PEEP(n+1) höher ist als der bisherige PEEP(n), so wird mit Vorteil ein Rekrutierungsmanöver (Block 108) durchgeführt. Dies bedeutet, dass der Druck angehoben und von einem erhöhten Druck zum PEEP(n+1) gelangt wird. Dies ist erforderlich, um alle Lungenbläschen, die unter diesem PEEP(n+1) offen bleiben, zu öffnen. Eine solche Rekrutierung kann auch durch ein P/V-Manöver erreicht werden. Ein gesondertes Rekrutierungsmanöver ist nicht erforderlich, wenn zeitgleich mit dem P/V-Manöver die Differenzbildung abläuft und der Druck, der bei der maximalen Volumendifferenz (oder in einem bestimmten Abstand vom Maximum) herrscht, als PEEP genommen und nicht unterschritten wird.

Danach kann mit dem neuen PEEP(n+1) beatmet werden (Block 109).

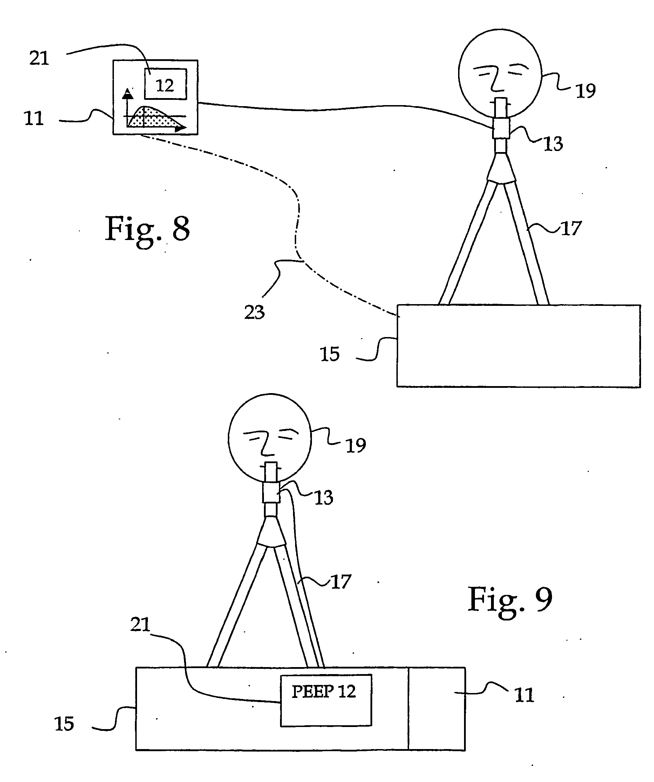

Die in Figur 8 dargestellte. Vorrichtung 11 ist so ausgerüstet, dass sie den PEEP erfindungsgemäss bestimmen kann. Sie besitzt einen Fluss/Drucksensor 13, mit dem in an sich bekannter Weise Druck und Volumen gemessen werden kann. Dieser ist z.B. patientenseitig an einem Beatmungsschlauch 17 angeordnet oder im Gerät integriert. Durch diesen Beatmungsschlauch wird der Patient 19 mittels des Beatmungsgeräts 15 beatmet.

Die Vorrichtung hat einen Display 21, auf dem der ermittelte Wert für PEEP angezeigt ist. Für die Ermittlung des angemessenen PEEP wird mit dem Beatmungsgerät 15 der Beatmungsdruck erhöht und wieder erniedrigt. Druck und Volumen werden im Sensor 13 gemessen und mit der Elektronik der Vorrichtung daraus der geeignete PEEP errechnet.

Durch die unterbrochene Linie 23 wird eine optionale Verbindung zwischen der erfindungsgemässen Vorrichtung und dem Beatmungsgerät dargestellt. Über diese Verbindung 23 kann der Wert für PEEP direkt in das System des Beatmungsgeräts eingegeben und/ oder ein P/V-Manöver ausgelöst werden.

In Figur 9 ist die erfindungsgemässe Vorrichtung 11 im Beatmungsgerät 15 integriert. Für die Erstellung der P/V-Schlaufe wird die Druckquelle des Beatmungsgeräts 15 und der Druck-/ Flusssensor 13 des Beatmungsgeräts genutzt. Der Wert für PEEP kann auf dem Display 21 angezeigt werden, und/oder automatisiert bei der Beatmung verwendet werden.

## Patentansprüche

1. Vorrichtung zum automatisierten Ermitteln eines auf einen Patienten abgestimmten PEEP,
- welche Vorrichtung Sensoren (13) und eine geeigneten Elektronik umfasst, um, während der Steuerung der Druckrampe des Beatmungsdrucks, vorzugsweise während der Durchführung eines P/V-Manövers, eine Druck-Volumen-Kurve ermitteln zu können,
- welche Elektronik ausgelegt ist, um beatmungsdruckspezifisch eine Differenzkurve der Volumina bei der Deflation und bei der Insufflation (Vdef-Vinf) zu bilden,
- ein Maximum dieser Differenzkurve zu suchen,
- einen Wert des Beatmungsdrucks bei einem in Relation zum Maximum definierten Volumen der Differenzkurve zu ermitteln,
- und aufgrund dieses ermittelten Werts des Beatmungsdrucks einen Wert für PEEP zu bestimmen.

2. Vorrichtung nach Anspruch 1, **gekennzeichnet durch** einen Display (21) zur Anzeige des ermittelten Werts für PEEP.

3. Vorrichtung nach Anspruch 1 oder 2, **gekennzeichnet durch** eine elektronische Verbindung (23) zu einem Beatmungsgerät (15) zur Übertragung des ermittelten Werts für PEEP und/ oder zur Auslösung eines P/V-Manövers.

4. Vorrichtung nach Anspruch 3, **gekennzeichnet durch** eine elektronische Verbindung (23) zur Steuerung eines Beatmungsgeräts (15), indem das Beatmungsgerät (15) den ermittelten Wert des Beatmungsdrucks automatisch als PEEP der Beatmung verwendet.

5. Vorrichtung nach einem der vorangehenden Ansprüche, **dadurch gekennzeichnet, dass** ein Grenzwert für die Volumendifferenz festgelegt ist, und die Elektronik nur dann einen erhöhten PEEP ermittelt, wenn das Maximum (Vdef-Vinf)max der Volumendifferenz oberhalb dieses Grenzwerts liegt.

6. Vorrichtung nach einem der vorangehenden Ansprüche, **dadurch gekennzeichnet, dass** Mittel vorhanden sind, um einem Krankheitsbild und/ oder einem Körperindex des Patienten entsprechend unterschiedliche Zahlenwerte aus einer Tabelle zu wählen oder zu berechnen, und die Elektronik derart ausgelegt ist, dass sie zur Bestimmung des PEEP diesen Wert des Beatmungsdrucks mit dem durch erwähnte Mittel gewählten oder berechneten Zahlenwert verrechnet.

7. Vorrichtung nach einem der vorangehenden Ansprüche, **dadurch gekennzeichnet, dass** der dem PEEP zugrunde zu legende Beatmungsdruck einer der Drücke unter dem beim Maximum herrschenden Druck , insbesondere der tiefste der Drücke ist, die an den Stellen herrschen, an denen die Differenzkurve einen um einen definierten Betrag unter dem Maximum liegenden Volumenwert aufweist.

8. Vorrichtung nach Anspruch 7, **dadurch gekennzeichnet, dass** dieser Betrag aufgrund des Grenzwerts gemäss Anspruch 6, oder in Abhängigkeit eines Körperindexes des Patienten und/oder seines Krankheitsbildes bemessen ist.

9. Beatmungsgerät (15) umfassend eine Vorrichtung (11) gemäss einem der vorangehenden Ansprüche.

## Claims

1. A Device for automatically determining a PEEP adjusted to a patient,
- said device comprising sensors (13) and electronics for being able to determine a pressure/volume curve during the control of the pressure ramp of the ventilation pressure, preferable during the implementation of a P/V-maneuver,
- said electronics designed to form a difference curve of the volumes during deflation and insufflations (Vdef-Vinf) in a manner specific to the ventilation pressure,
- to search for a maximum of this difference curve,
- to determine a value of the ventilation pressure at a volume of the difference curve defined in relation to the maximum,
- and to determine a value for PEEP on the basis of this determined value of the ventilation pressure.

2. The device according to claim 1, **characterized by** a display (21) for displaying the values determined for PEEP.

3. The device according to claim 1 or 2, **characterized by** an electronic connection (23) to a ventilation device (15) for transmitting the value determined for PEEP and/or for triggering a P/V-maneuver.

4. The device according to claim 3, **characterized by** an electronic connection (23) for controlling a ventilation device (15), in such a manner that the ventilation device (15) automatically uses the value determined for the ventilation pressure as PEEP for the ventilation.

5. The device according to any one of prior claims, **characterized in that**, a limit value for the volume difference is established, and the electronics only determine an increased PEEP if the maximum (Vdef-Vinf)max of the volume difference lies above this limit value.

6. The device according to any one of prior claims, **characterized in that**, means are present for selecting numerical values from a table or calculating them, in accordance with a disease profile and/or a body index of the patient, and the electronics are designed in such a manner that they process this value of the ventilation pressure with the numerical value selected or calculated using the aforementioned means, in order to determine the PEEP.

7. The device according to any one of prior claims, **characterized in that**, the ventilation pressure on which the PEEP is based is one of the pressures below the pressure prevailing at the maximum, in particular is the slowest of the pressures, that prevail at locations at which the difference curve has a volume value that lies a defined amount below the maximum.

8. The device according to claim 7, **characterized in that**, this amount is established on the basis of the limit value according to claim 6, or as a function of the body index of the patient and/or the disease profile of the patient.

9. Ventilation device (15) comprising a device (11) according to any one of the prior claims.

## Revendications

1. Dispositif pour la détermination automatisée d'une PEEP adaptée à un patient,
- lequel dispositif comprend des capteurs (13) et une électronique appropriée pour pouvoir déterminer, pondant la commande de la rampe de pression de la pression de ventilation, de préférence pendant l'exécution d'une manoeuvre de PN, une courbe de pression/volume,
- laquelle électronique est dimensionnée pour former, de manière spécifique à la pression de ventilation, une courbe de différence des volumes lors de l'expiration et lors de l'insufflation (Vdef-vinf),
- pour chercher un maximum de cette courbe de différence,
- pour déterminer une valeur de la pression de ventilation pour un volume de la courbe de différence qui est défini en relation avec le maximum
- et pour déterminer une valeur pour la PEEP en raison de cette valeur déterminée de la pression de ventilation.

2. Dispositif selon la revendication 1, **caractérisé par** un affichage (21) pour afficher la valeur déterminée pour la PEEP.

3. Dispositif selon la revendication 1 ou 2, **caractérisé par** une connexion électronique (23) à un appareil de ventilation (15) pour la transmission de la valeur déterminée pour la PEEP et/ou pour le déclenchement d'une manoeuvre P/V.

4. Dispositif selon la revendication 3, **caractérisé par** une connexion électronique (23) pour la commande d'un appareil de ventilation (15), l'appareil de ventilation (15) utilisant la valeur déterminée de l'appareil de ventilation automatiquement comme PEEP de la ventilation.

5. Dispositif selon l'une des revendications précédentes, **caractérisé en ce qu'**une valeur de seuil est fixée pour la différence de volume et l'électronique ne détermine une PEEP accrue que si le maximum (Vdef-Vinf)max de la différence de volume est situé au-dessus de cette valeur de seuil.

6. Dispositif selon l'une des revendications précédentes, **caractérisé en ce qu'**il existe des moyens pour sélectionner ou calculer un tableau clinique et/ou un indice corporel du patient selon différentes valeurs numériques d'un tableau et que l'électronique est dimensionnée telle qu'elle combine, pour déterminer la PEEP, cette valeur de la pression de ventilation à la valeur numérique sélectionnée ou calculée par les moyens mentionnés.

7. Dispositif selon l'une des revendications précédentes, **caractérisé en ce que** la pression de ventilation sur laquelle la PEEP doit être basée est l'une des pressions en dessous de la pression qui prévaut au maximum, en particulier la plus basse des pressions qui prévalent aux endroits auxquels la courbe de différence présente une valeur de volume située d'une certaine valeur en dessous du maximum.

8. Dispositif selon la revendication 7, **caractérisé en ce que** cette valeur est dimensionnée en raison de la valeur de seuil selon la revendication 6 ou en fonction d'un indice corporel du patient et/ou de son tableau clinique.

9. Appareil de ventilation (15) comportant un dispositif (11) selon l'une des revendications précédentes.
